(19) Europäisches Patentamt
European Patent Office
Office européen des brevets

(11) Publication number: **0 057 564 A1**

# (12) EUROPEAN PATENT APPLICATION

(21) Application number: **82300406.4**

(22) Date of filing: **27.01.82**

(51) Int. Cl.³: **C 07 D 249/14**, C 07 D 405/12, C 07 C 127/26, C 07 D 307/52, C 07 D 295/08 // A61K31/41, A61K31/445, A61K31/55

(30) Priority: **30.01.81 US 230134**

(43) Date of publication of application: **11.08.82 Bulletin 82/32**

(84) Designated Contracting States: **AT BE CH DE FR GB IT LI LU NL SE**

(71) Applicant: **SMITHKLINE CORPORATION, 1500 Spring Garden Street P.O. Box 7929, Philadelphia Pennsylvania 19101 (US)**

(72) Inventor: **Webb, Robert Lee, 512 N. Veronica Road, West Chester Pennsylvania 19380 (US)**

(74) Representative: **Waters, David Martin, Dr. et al, Smith Kline & French Laboratories Ltd. Patent Department Mundells, Welwyn Garden City Hertfordshire AL7 1EY (GB)**

(54) **Process for preparing 3,5-diamino-1,2,4-triazoles.**

(57) 3,5-Diamino-1,2,4-triazoles

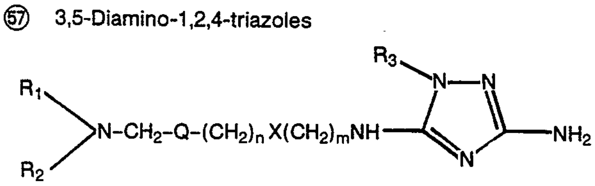

in which $R_1$ and $R_2$ are each lower alkyl or form part of a pyrrolidinyl, piperidinyl or hexamethyleneiminyl ring; Q is a furan or benzene ring; n is 0 or 1; m is 2 or 3; X is sulphur when n is 1, m is 2, and Q is furan, and X is oxygen when n is 0, m is 3 and Q is benzene; and $R_3$ is hydrogen or lower alkyl are prepared by reacting an O-phenyl isourea

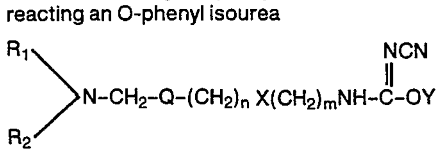

in which Y is phenyl and $R_1$, $R_2$, Q, n, m and X are as defined above, with a hydrazine $NH_2NHR_3$ in which $R_3$ is as defined above.

EP 0 057 564 A1

# PROCESS FOR PREPARING 3,5-DIAMINO-1,2,4-TRIAZOLES

This invention relates to a new process for preparing 3,5-diamino-1,2,4-triazoles using diphenyl N-cyanoimidocarbonate as a starting material. In addition, this invention relates to new intermediates in this process.

The 3,5-diamino-1,2,4-triazoles which may be prepared by the process of this invention have been described as having histamine $H_2$-receptor blocking activity in U.K. Patent Application GB 2,023,133A, published December 28, 1979. In particular, the following 3,5-diamino-1,2,4-triazoles which are described therein are prepared by the process of this invention:

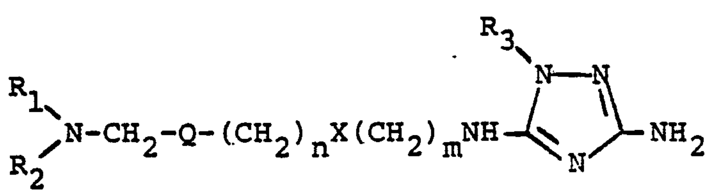

FORMULA I

in which:

$R_1$ and $R_2$ are each lower alkyl or $R_1$ and $R_2$ may, together with the nitrogen atom to which they are attached, form a pyrrolidinyl, piperidinyl or hexamethyleneiminyl ring;

Q is a furan or benzene ring;

n is 0 or 1;

m is 2 or 3;

X is sulphur when n is 1, m is 2 and Q is furan, and X is oxygen when n is 0, m is 3 and Q is benzene; and

$R_3$ is hydrogen or lower alkyl.

As disclosed in U.K. Patent Application GB 2,023,133A, the compounds of Formula I have pharmacological activity as selective histamine $H_2$-antagonists, and compounds with histamine $H_2$-blocking activity may be used in the treatment of conditions where there is a hypersecretion of gastric acid, and in the treatment of allergic and inflammatory conditions where histamine is a mediator.

Several methods of preparing 3,5-diamino-1,2,4-triazoles are disclosed in G.B. 2,023,133 A, one of them being the reaction of a hydrazine with a compound of the formula:

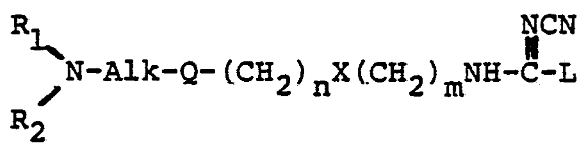

in which L is defined as a leaving group, for example a lower alkoxy or lower alkylthio group.

According to the process of this invention, 3,5-diamino-1,2,4-triazoles are prepared by a procedure in which diphenyl N-cyanoimidocarbonate is reacted with a substituted amine and the resulting O-phenyl isourea intermediate is reacted with a hydrazine. The process of this invention provides high yields of the 3,5-diamino-1,2,4-triazoles. It is advantageous over the process in which L is lower alkylthio because it avoids the toxic and malodorous mercaptans evolved in that process. In the process of this invention, the leaving group is phenoxy which is a superior leaving group (L) and is advantageous over lower alkoxy since the reaction can be carried out in high yield under mild conditions. Also, the intermediates with L = phenoxy are more stable than the lower alkoxy analogues.

The process of this invention is represented as follows:

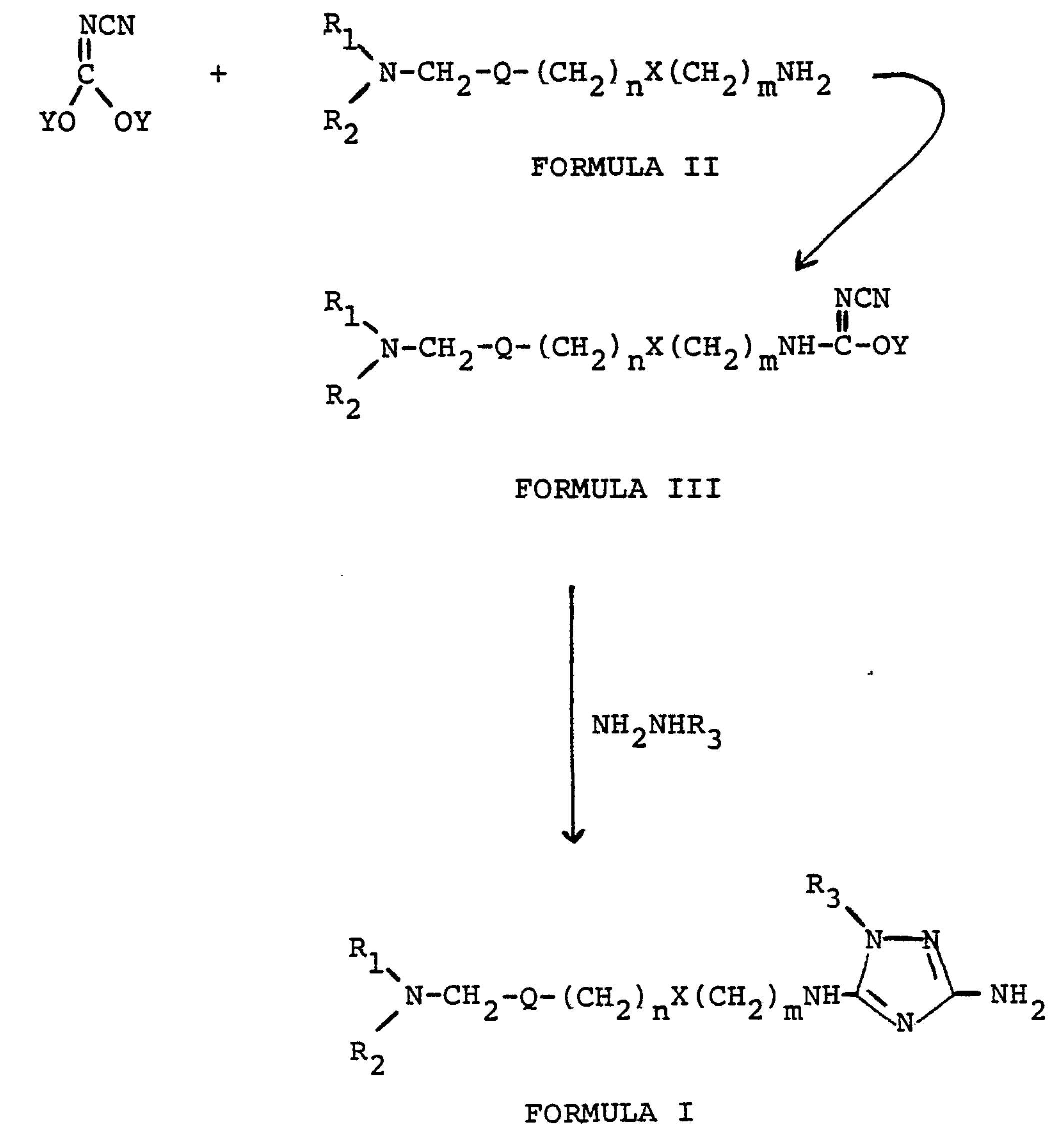


FORMULA II

FORMULA III

FORMULA I

The term Y is phenyl and $R_1$, $R_2$, Q, n, m, X and $R_3$ are as defined above.

The diphenyl N-cyanoimidocarbonate is reacted with an equimolar amount of an amine of Formula II in an inert solvent such as ethyl acetate, isopropanol or other alcohols, tetrahydrofuran, dioxan, acetonitrile or preferably ethyl ether at about room temperature to give the O-phenyl isourea intermediate (Formula III). These O-phenyl isoureas are the new intermediate compounds of this invention.

The O-phenyl isourea is reacted with a hydrazine in an inert solvent at elevated temperature, for example in ethyl ether or isopropanol at reflux temperature, to give the 3,5-diamino-1,2,4-triazoles of Formula I.

Alternatively, to prepare the compounds in which $R_3$ is lower alkyl, the O-phenyl isourea may be reacted with a N-lower alkylhydrazine having a N'-protecting group such as a N'-phenylmethylene group. The reaction may be carried out in an inert solvent such as isopropanol at about room temperature, followed by treatment with dilute hydrochloric acid to remove the protecting group and cyclize to give the 3,5-diamino-1,2,4-triazoles.

Also, the diaminotriazoles may be prepared by reacting diphenyl N-cyanoimidocarbonate first with a N-lower alkyl-N'-protected hydrazine, then reacting the resulting intermediate with an amine of Formula II and treating the resulting intermediate with dilute hydrochloric acid to give the 3,5-diamino-1,2,4-triazoles.

The diphenyl N-cyanoimidocarbonate starting material is prepared by the following procedure:

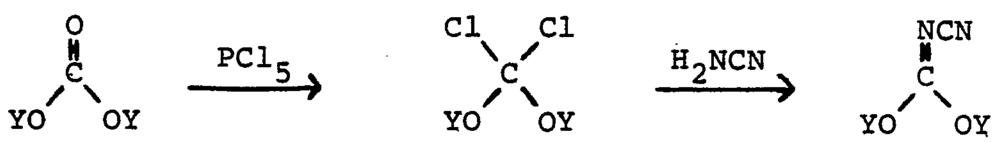


in which Y is phenyl. According to this procedure, diphenyl carbonate is reacted with phosphorus pentachloride at 150-160° to form 1,1-dichloro-1,1-diphenoxymethane which is reacted with cyanamide in an inert organic solvent at temperatures below 80°, preferably at room temperature.

All temperatures herein are in degrees Centigrade.

The invention is illustrated but not limited by the following examples.

EXAMPLE 1

To a suspension of 24.0 g. (0.2 mol) of diphenyl N-cyanoimidocarbonate in 250 ml. of ether was added 22.0 g. (0.2 mol) of 2-(5-dimethylaminomethyl-2-furylmethylthio)ethylamine at such a rate that the ether refluxed gently. The solution was stirred for one hour at room temperature and the white precipitate which formed was filtered, washed with 50 ml. ether and dried to give N-cyano-N'-[2-(5-dimethylaminomethyl-2-furylmethylthio)-ethyl]-O-phenylisourea, m.p. 74-75$^o$, yield 34.2 g. (95%).

To a stirred solution of 3.6 g. (0.01 mol) of N-cyano-N'-[2-(5-dimethylaminomethyl-2-furylmethylthio)ethyl]-O-phenylisourea in 50 ml. of isopropanol was added 0.32 g. (0.01 mol) of hydrazine and the solution was refluxed for one hour. The isopropanol was concentrated and the resultant precipitate filtered and recrystallized from water to give $N^5$-[2-(5-dimethylaminomethyl-2-furylmethylthio)ethyl]-1H-1,2,4-triazole-3,5-diamine, m.p. 77-79$^o$, yield, 2.37 g. (80%).

EXAMPLE 2

N-methylhydrazine (0.46 g., $10^{-2}$ mol) was added to a stirred solution of N-cyano-N'-[2-(5-dimethylaminomethyl-2-furylmethylthio)ethyl]-O-phenylisourea (3.59 g., $10^{-2}$ mol) in 65 ml. of ethyl acetate and the mixture stirred at room temperature overnight (14 hours). The mixture was then refluxed for one hour, cooled and the ethyl acetate evaporated. The residue was dissolved in ethanol and treated with excess oxalic acid. The precipitate was collected, washed with a small quantity of ethanol and dried to give 1-methyl-$N^5$-[2-(5-dimethylaminomethyl-2-furylmethylthio)ethyl]-1H-1,2,4-triazole-3,5-diamine, m.p. 162-163$^o$ (dec.), yield 2.0 g. (50%).

EXAMPLE 3

To a stirred suspension of 2.3 g. (.0096 mol) of diphenyl N-cyanoimidocarbonate in 40 ml. of ether was added 2.0 g. (.0096 mol) of 3-[3-(dimethylaminomethyl)-phenoxy]propylamine and the mixture was stirred for one hour at room temperature. The white precipitate was filtered and washed with ether to yield after drying N-cyano-N'-[3-(3-dimethylaminomethylphenoxy)propyl]-O-phenylisourea, m.p. 99-100$^{o}$, yield 3.1 g. (92%).

To a suspension of 1.0 g. (2.8 x $10^{-3}$ mol) of N-cyano-N'-[3-(3-dimethylaminomethylphenoxy)propyl]-O-phenylisourea in ether was added 0.131 g. (2.8 x $10^{-3}$ mol) of N-methylhydrazine and the mixture was stirred for one hour at room temperature and then refluxed for one hour. The ether was removed and the yellow oil chromatographed on silica using chloroform/methanol as eluent. Recrystallization of the appropriate fraction from petroleum ether/ethyl ether and drying gave 1-methyl-$N^5$-[3-(3-dimethylaminomethylphenoxy)propyl]-1H-1,2,4-triazole-3,5-diamine, m.p. 95-97$^{o}$, yield 610 mg. (72%).

EXAMPLE 4

Reacting N-cyano-N'-[3-(3-dimethylaminomethyl-phenoxy)propyl]-O-phenylisourea with hydrazine in ether at room temperature and working up by the procedure of Example 1 gives $N^5$-[3-(3-dimethylaminomethylphenoxy)-propyl]-1H-1,2,4-triazole-3,5-diamine.

EXAMPLE 5

By the procedure of Example 3, reacting diphenyl N-cyanoimidocarbonate with the following amines:

a. 3-[3-(1-piperidinylmethyl)phenoxy]propylamine
b. 3-[4-(1-piperidinylmethyl)phenoxy]propylamine
c. 3-[3-(1-pyrrolidinylmethyl)phenoxy]propylamine
d. 3-[3-(1-hexamethyleneiminylmethyl)phenoxy]-propylamine

the following O-phenyl isourea intermediates are prepared:

a. N-cyano-N'-[3-(3-(1-piperidinylmethyl)-phenoxy)propyl]-O-phenylisourea

b. N-cyano-N'-[3-(4-(1-piperidinylmethyl)-phenoxy)propyl]-O-phenylisourea

c. N-cyano-N'-[3-(3-(1-pyrrolidinylmethyl)-phenoxy)propyl]-O-phenylisourea

d. N-cyano-N'-[3-(1-hexamethyleneiminylmethyl)-phenoxy)propyl]-O-phenylisourea

Reacting these intermediates with N-methylhydrazine by the procedure of Example 3 gives:

a. 1-methyl-$N^5$-[3-(3-1-piperidinylmethyl) phenoxy)propyl]-1H-1,2,4-triazole-3,5-diamine

b. 1-methyl-$N^5$-[3-(4-1-piperidinylmethyl)-phenoxy)propyl]-1H-1,2,4-triazole-3,5-diamine

c. 1-methyl-$N^5$-[3-(3-1-pyrrolidinylmethyl)-phenoxy)propyl]-1H-1,2,4-triazole-3,5-diamine

d. 1-methyl-$N^5$-[3-(3-(1-hexamethyleneiminylmethyl)phenoxy)propyl]-1H-1,2,4-triazole-3,5-diamine.

EXAMPLE 6

A mixture of N-cyano-N'-[2-(5-dimethylaminomethyl-2-furylmethylthio)ethyl]-O-phenylisourea and N-ethylhydrazine in isopropanol is heated at reflux for one hour to give, after working up by the procedure of Example 2, 1-ethyl-N -[2-(5-dimethylaminomethyl-2-furylmethylthio)-ethyl]-1H-1,2,4-triazole-3,5-diamine.

By the same procedure, using N-propylhydrazine and N-butylhydrazine, the corresponding 1-propyl and 1-butyl compounds are prepared.

EXAMPLE 7

By the procedure of Example 1, reacting diphenyl N-cyanoimidocarbonate with 2-[5-(1-piperidinylmethyl)-2-furylmethylthio]ethylamine gives N-cyano-N'-[2-(5-(1-piperidinylmethyl)-2-furylmethylthio)ethyl]-O-phenylisourea. This phenylisourea is reacted with N-methylhydrazine by the procedure of Example 2 to give 1-methyl-$N^5$-[2-(5-(1-piperidinylmethyl)-2-furylmethylthio)ethyl]-1H-1,2,4-triazole-3,5-diamine.

Using hydrazine, instead of N-methylhydrazine, there is obtained by the procedure of Example 1, $N^5$-[2-(5-(1-piperidinylmethyl)-2-furylmethylthio)ethyl]-1H-1,2,4-triazole-3,5-diamine.

Also, using 2-[5-(1-pyrrolidinylmethyl)-2-furylmethylthio]ethylamine in the above procedure, N-cyano-N'-[2-(5-(1-pyrrolidinylmethyl)-2-furylmethylthio)ethyl]-O-phenylisourea is obtained and then is reacted with N-methylhydrazine to give 1-methyl-$N^5$-[2-(5-(1-pyrrolidinylmethyl)-2-furylmethylthio)ethyl]-1H-1-1,2,4-triazole-3,5-diamine.

What is claimed is:

1. A process for preparing 3,5-diamino-1,2,4-triazoles of the formula:

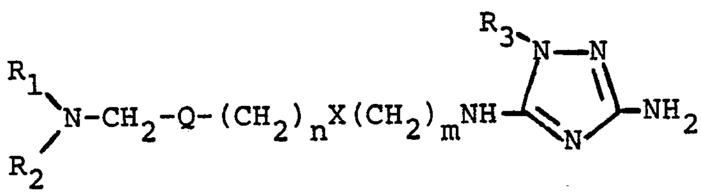

in which:

$R_1$ and $R_2$ are each lower alkyl or $R_1$ and $R_2$ may, together with the nitrogen atom to which they are attached, form a pyrrolidinyl, piperidinyl or hexamethyleneiminyl ring;

Q is a furan or benzene ring;

n is 0 or 1;

m is 2 or 3;

X is sulphur when n is 1, m is 2 and Q is furan, and X is oxygen when n is 0, m is 3 and Q is benzene; and

$R_3$ is hydrogen or lower alkyl

which comprises reacting an O-phenyl isourea of the formula:

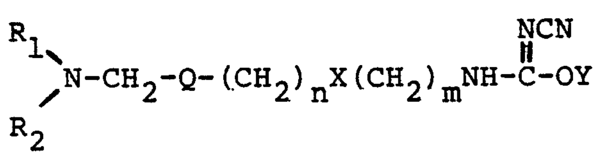

in which Y is phenyl and $R_1$, $R_2$, Q, n, m and X are as defined above, with a hydrazine of the formula:

$$NH_2NHR_3$$

in which $R_3$ is as defined above.

2. A process according to claim 1 in which the O-phenyl isourea and the hydrazine are reacted in an inert solvent at elevated temperature.

3. A process according to claim 2 in which the reaction is carried out in ethyl ether or isopropanol at reflux temperature.

4. A process according to claim 1 characterized in that the O-phenyl isourea intermediate of the formula:

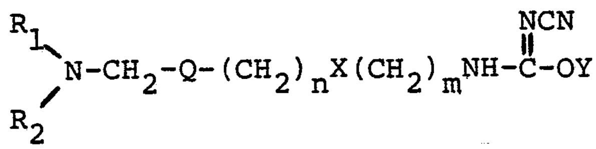

in which:

$R_1$ and $R_2$ are each lower alkyl or $R_1$ and $R_2$ may, together with the nitrogen atom to which they are attached, form a pyrrolidinyl, piperidinyl or hexamethyleneiminyl ring;

Q is a furan or benzene ring;

n is 0 or 1;

m is 2 or 3;

X is sulphur when n is 1, m is 2 and Q is furan, and X is oxygen when n is 0, m is 3 and Q is benzene; and

Y is phenyl

is prepared by reacting diphenyl N-cyanoimidocarbonate of the formula:

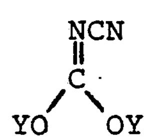

in which Y is as defined above, with an amine of the formula:

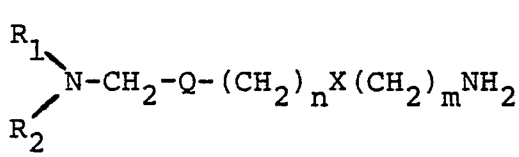

in which $R_1$, $R_2$, Q, n, m and X are as defined above.

5. A process according to claim 4 in which diphenyl N-cyanoimidocarbonate and the amine are reacted in an inert solvent at about room temperature.

6. A process according to claim 5 in which the inert solvent is ethyl ether.

7. A compound of the formula:

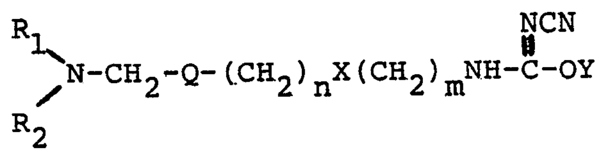

in which:

$R_1$ and $R_2$ are each lower alkyl or $R_1$ and $R_2$ may, together with the nitrogen atom to which they are attached, form a pyrrolidinyl, piperidinyl or hexamethyleneiminyl ring;

Q is a furan or benzene ring;

n is 0 or 1;

m is 2 or 3;

X is sulphur when n is 1, m is 2 and Q is furan, and X is oxygen when n is 0, m is 3 and Q is benzene; and

Y is phenyl.

8. A compound according to claim 7 which is N-cyano-N'-[2-(5-dimethylaminomethyl-2-furylmethylthio)-ethyl]-O-phenylisourea.

9. A compound according to claim 7 which is N-cyano-N'-[3-(3-dimethylaminomethylphenoxy)propyl]-O-phenylisourea.

European Patent Office

**EUROPEAN SEARCH REPORT**

0057564

Application number
EP 82300406.4

## DOCUMENTS CONSIDERED TO BE RELEVANT

| Category | Citation of document with indication, where appropriate, of relevant passages | Relevant to claim |
|---|---|---|
| A | DE - A1 - 2 917 026 (GLAXO) * Claim 1; pages 28-31, 56-59 * | 1,4,7 |
| D,A | & GB-A-2 023 133 | |
| | -- | |
| A | EP - A1 - 0 007 232 (SMITH KLINE) * Claim 11 * | 4 |
| | -- | |
| X | DE - A1 - 2 734 070 (ALLEN) * Page 22, formula III; claims 1,37b * | 7,8 |
| | ---- | |

**CLASSIFICATION OF THE APPLICATION (Int. Cl. 3)**

C 07 D 249/14
C 07 D 405/12
C 07 C 127/26
C 07 D 307/52
C 07 D 295/08//
A 61 K 31/41
A 61 K 31/445
A 61 K 31/55

**TECHNICAL FIELDS SEARCHED (Int.Cl. 3)**

C 07 D 249/00
C 07 D 405/00
C 07 C 127/00
C 07 D 307/00
C 07 D 295/00
C 07 D 239/00

**CATEGORY OF CITED DOCUMENTS**

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

X The present search report has been drawn up for all claims

| Place of search | Date of completion of the search | Examiner |
|---|---|---|
| VIENNA | 20-04-1982 | BRUS |